# EUROPEAN PATENT APPLICATION

(11) **EP 4 520 669 A1**
(43) Date of publication of application: **12.03.2025**
(21) Application number: 23195600.4
(22) Date of filing: 06.09.2023
(51) Int. Cl.: B64U 10/13, A61B 34/32, B64U 80/25

(54) **UNMANNED AERIAL VEHICLE FOR MONITORING A MEDICAL IMAGING SYSTEM, MONITORING SYSTEM AND METHOD**

(71) Applicant: Koninklijke Philips N.V., 5656 AG Eindhoven (NL)
(72) Inventor: WEISS, Steffen, 5656 AG Eindhoven (NL)
(74) Representative: Philips Intellectual Property & Standards

(57) **Abstract**

The invention relates to an unmanned aerial vehicle (1) for monitoring a medical imaging system (8). The unmanned aerial vehicle (1) comprises an aerodynamic arrangement (2), a navigation sensor (3), a computing unit (4), a monitoring sensor and a memory (6) and/or a transmitter (7). The computing unit (4) is configured to receive output from the navigation sensor (3) and to control the aerodynamic arrangement (2) based on said output from the navigation sensor (3). The monitoring sensor (5) is configured to acquire monitoring data, the memory (6) is configured to store the monitoring data or data based on the monitoring data and the transmitter (7) is configured to wirelessly transmit the monitoring data or data based on the monitoring data. The invention further relates to a monitoring system (1; 14) comprising the unmanned aerial vehicle (1) and a base station (14) as well a to a method for operating the unmanned aerial vehicle (1).

## Description

### FIELD OF THE INVENTION

The invention relates to medical imaging sytems and, in particular, to monitoring of such medical imaging systems. More particularly, the invention relates to an unmanned aerial vehicle for monitoring a medical imaging system, to a monitoring system with said unmanned aerial vehicle and to a method for operating said unmanned aerial vehicle.

### BACKGROUND OF THE INVENTION

Medical imaging requires fast, work-efficient, safe, and correct positioning of patients, sensors, and auxiliary equipment as part of the patient preparation. These tasks are typically performed by staff in the current clinical routine, but may also be performed automatically using cameras and other sensors, e.g., to detect and check the patient position and pose. Such sensors can also be used to measure vital signs or estimate patient data such as weight or size.

Depending on the specific monitoring task, different positions of the sensors may be needed. As an example, for patient preparation, the camera may have to be positioned at the ceiling above the center of a patient table. This, however, causes several problems: For example, medical imaging suites are not standardized so that the ceiling type and height are different for each suite. Hence, custom solutions for position, fixation, and wiring are needed for each system installation. Further, the ceiling above the table may already be used for other purposes such as room lighting, projectors, or medical equipment. Therefore, simple and fast installations of monitoring cameras as well as simple and fast upgrades of monitoring camera are impossible.

Alternatively, the camera may be fixed at an arm extending from the imaging system. However, this solution is not robust and requires space close to the imaging system.

Moreover, a fixed camera can observe the patient only out of one perspective, which may be suboptimal for tasks requiring different detection positions and/or the detection of auxiliary equipment at locations other than on the patient table.

### SUMMARY OF THE INVENTION

It is therefore an object of the present invention to provide a monitoring device, a related monitoring system and a method for operation said monitoring device that overcome the above-mentioned problems, in particular that allow variable positioning of monitoring sensor for monitoring a medical imaging system.

The object of the present invention is solved by the subject matter of the independent claims, wherein further embodiments are incorporated in the dependent claims.

In an aspect of the present invention, an unmanned aerial vehicle for monitoring a medical imaging system is provided. In this context, unmanned aerial vehicle may refer to any remotely controlled and/or wirelessly controlled aerial vehicle, such as a drone, an unmanned helicopter, unmanned quadcopter and/or an unmanned multicopter.

The medical imaging system may refer to any system with a medical imaging device such as a digital X-ray (DXR) imaging device, computed tomography (CT) imaging device and/or magnetic resonance imaging (MRI) device. Besides the medical imaging device, the medical imaging system may comprise a medical imaging room, e.g., an MRI room or a CT room, a technical room, auxiliary devices, a patient table and/or a patient.

The unmanned aerial vehicle comprises an aerodynamic arrangement, a navigation sensor, a computing unit, a monitoring sensor and a memory and/or a transmitter.

The aerodynamic arrangement may be configured to propel and steer the unmanned aerial vehicle.

The navigation sensor may be configured to obtain navigation data that is used to determine the position of the unmanned aerial vehicle, in particular with respect to a predefined reference location.

The computing unit is configured to receive output from the navigation sensor and to control the aerodynamic arrangement based on said output from the navigation sensor. In particular, the computing unit is configured control the aerodynamic arrangement such that the unmanned aerial vehicle is steered to a desired location. In this context, the desired location may be a predefined location, one out of plurality of predefined locations, or a location provided by user input.

The monitoring sensor is configured to acquire monitoring data. In particular, the unmanned aerial vehicle may comprise a plurality of monitoring sensors in order to monitor a plurality of parameters. It is possible that a single sensor serves as both a navigation sensor and a monitoring sensor, e.g., a camera may be used for computer vision for navigating and for monitoring purposes.

The memory is configured to store the monitoring data or data based on the monitoring data. That is, the memory may store the direct output from the monitoring sensor. Alternatively, or additionally, the output from the monitoring sensor may be processed and the memory may store said processed output, which is data based on the monitoring data. Said processing may be performed by a processing unit allocated to the monitoring sensor or by the computing unit.

The transmitter is configured to wirelessly transmit the monitoring data or data based on the monitoring data. Said transmitting may be to a dedicated wireless receiver or to a standard wireless receiver, e.g., of the medical imaging system. The transmitted data may then be used by a monitoring device that, e.g., outputs the data to a user. Alternatively, or additionally, the transmitted data may be used by the medical imaging system. Again, the transmitter may transmit the direct output from the monitoring sensor. Alternatively, or additionally, the output from the monitoring sensor may be processed and the transmitter may transmit said processed output, which is data based on the monitoring data. Said processing may be performed by a processing unit allocated to the monitoring sensor or by the computing unit.

It is also possible that the unmanned aerial vehicle comprises both a memory and a transmitter. In this case, some of the monitoring data or of the data based on the monitoring data may be stored in the memory and other monitoring data or data based on the monitoring data may be wirelessly transmitted.

The above-described unmanned aerial vehicle requires little effort for installation and/or a system upgrade. Further, the unmanned aerial vehicle can be controlled very precisely and can be steered to almost arbitrary locations of interest to provide one or more optimal views of the patient and/or other parts of the medical imaging system.

The unmanned aerial vehicle may further comprise a receiver configured to wirelessly receive commands. Said commands may be, in particular, steering and monitoring commands, e.g., to steer the unmanned aerial vehicle to a location of interest and perform specific monitoring at said location of interest. The commands may be provided to a wireless transmitter, e.g., a dedicated wireless transmitter or a wireless transmitter of the medical imaging system, either by user input or automatically, e.g., by the medical imaging system.

According to an embodiment, the aerodynamic arrangement comprises at least one rotating part and at least one motor, wherein the motor is configured to actuate the rotating part. In particular, the rotating part may be a rotor of a helicopter or a multicopter. The motor may be an electric motor. For medical imaging systems that have strong magnetic fields, electric motors without permanent magnets may be used. In order to minimize the effect of the magnetic field on the motor, a piezo motor, an ultrasonic motor, or a stepper motor may be used. Alternatively, or additionally, the motor may be a compressed air motor or another motor using at least mostly mechanical energy, which are even less prone to effects of strong magnetic fields and also do not create magnetic fields themselves.

According to an embodiment, the navigation sensor is a camera. The images captured by said camera may be used to detect landmarks, e.g., specifically installed landmarks or characteristic features of the medical imaging device, for example, the patient table or an outline of an MRI bore, and the position of the unmanned aerial vehicle is determined based on said detected landmarks.

Alternatively, or additionally, the navigation sensor is an infrared sensor and/or a time-of-flight sensor. The navigation is then, again, based on landmarks detected with said sensors.

Alternatively, or additionally, the navigation sensor is a radio sensor. In particular, a plurality of radio transmitters may be installed, e.g., in the medical imaging room, and the radio sensor receives the signals from these radio transmitters. The location of the unmanned aerial vehicle is then determined based on time delays between the received radio signals.

According to an embodiment, the monitoring sensor is a camera. With said camera, a wide variety of monitoring may be performed. As an example, patient positioning may be monitored with the camera, vital signs of a patient may be monitored with the camera, and inspections of the medical imaging system may be performed with the camera, e.g., the state of parts of the medical imaging system may be inspected, and parts in the medical imaging room may be recognized to determine the completeness of said parts or to determine foreign parts.

Alternatively, or additionally, the monitoring sensor is a microphone. With said microphone, sounds produced by a patient, e.g., breathing sounds or articulation of the patient, and/or sounds generated by the medical imaging system may be observed.

Alternatively, or additionally, the monitoring sensor is a radiation sensor, a magnetic field sensor, a radio frequency sensor, a thermometer and/or a hygrometer. With these sensors, the medical imaging system may be controlled, e.g., it can be checked whether the acquired monitoring data is within the specifications of the medical imaging system.

Alternatively, or additionally, the monitoring sensor is a near field communication sensor. With said sensor, the unmanned aerial vehicle may obtain data from parts of the medical imaging system that are equipped to provide, e.g., status information, via near field communication.

According to another aspect of the invention, a monitoring system for monitoring a medical imaging system is provided. Said monitoring system comprises an unmanned aerial vehicle according to the above description and a base station.

The base station may be located on a top of a medical imaging device of the medical imaging system or in the vicinity of the medical imaging device. The location in the vicinity of the medical imaging device may be preferred over the location on top of the medical imaging device for an MRI system in order to avoid the strong magnetic fields that may be present at the top of the MRI device. The base station may provide a landing platform for the unmanned aerial vehicle. Further, the base station may be connected to a monitoring device that, e.g., outputs data to a user, to the medical imaging device and/or to the medical imaging system.

Said monitoring system requires little effort for installation and/or a system upgrade. Further, the unmanned aerial vehicle of the monitoring system can be controlled very precisely and can be steered to almost arbitrary locations of interest to provide one or more optimal views of a patient and/or other parts of the medical imaging system.

According to an embodiment, the base station is configured to provide energy to the unmanned aerial vehicle. In particular, the base station may be configured to provide electrical energy to the unmanned aerial vehicle, more particularly in order to recharge batteries of the unmanned aerial vehicle. The electrical energy may be transferred using a electrical connection or wirelessly. Said electrical energy may be used to run the sensors and the computing unit and, in case of an unmanned aerial vehicle with an electric motor, to run said electric motor. The base station may also be configured to provide other forms of energy to the unmanned aerial vehicle, e.g., compressed air.

According to an embodiment, the base station further comprises a receiver configured to receive wireless output from the unmanned aerial vehicle. That is, the base station may receive the monitoring data or data based on the monitoring data from the unmanned aerial vehicle, and forward said data, e.g., to the monitoring device, to the medical imaging device and/or the medical imaging system. Since the data is received wirelessly, it can be received in real-time, providing a real-time monitoring of the medical imaging system, e.g., the medical imaging device and/or the patient.

According to an embodiment, the base station is configured to transfer data from the memory of the unmanned aerial vehicle when the unmanned aerial vehicle is docked to the base station. Said data is transferred to the base station and may be forwarded to, e.g., the monitoring device, the medical imaging device and/or the medical imaging system. Transferring the data from the memory may be performed by establishing an electrical connection between the unmanned aerial vehicle and the base station and/or via near field communication. Hence, the data collected by the unmanned aerial vehicle may be used, and large amounts of data can be easily transferred.

Further, the embodiments described above for the unmanned aerial vehicle may be combined with the embodiments for the monitoring system.

According to yet another aspect of the present invention, a method for operating an unmanned aerial vehicle according to the above description is provided. In this context, operating may refer to remote controlled operating, e.g. via a base station, or to autonomous operating.

According to the method, the unmanned aerial vehicle takes off and then navigates to a monitoring location, i.e., to a location of interest. The monitoring location may be a predefined location, or may be one out of plurality of predefined locations. The unmanned aerial vehicle may also receive, via a wireless connection, commands indicating the monitoring location. Alternatively, or additionally, the unmanned aerial vehicle may have received information indicating the monitoring location before taking off.

Navigating the unmanned aerial vehicle may be performed, e.g., using camera vision, identifying landmarks such as specifically installed landmarks or landmarks of the medical imaging device such as a patient table or an outline of an MRI bore. Alternatively, or additionally, navigating the unmanned aerial vehicle may be performed using radio signals that are transmitted by radio transmitters that are installed at known positions. The time delays of said radio signals are then used to determine the position of the unmanned aerial vehicle.

Further, the unmanned aerial vehicle acquires monitoring data, in particular using the monitoring sensor(s) of the unmanned aerial vehicle. For this, the monitoring sensors may have to controlled, e.g., the camera has to be adjusted to point in the desired direction.

The acquired monitoring data may then be processed, e.g., by the computing unit of the unmanned aerial vehicle, to obtain data based on the monitoring data.

The monitoring data and/or the data based on the monitoring data is then stored in the memory of the unmanned aerial vehicle. From the memory, said data may be transferred, e.g., to a base station, afterwards. This allows for the exchange of large amounts of data.

Alternatively, or additionally, the monitoring data and/or the data based on the monitoring data is wirelessly transmitted, e.g., to the base station, to the medical imaging device and/or to the medical imaging system. This enables real-time analysis of the acquired monitoring data.

Finally, the unmanned aerial vehicle lands, e.g., on a landing platform of the base station.

By operating the unmanned aerial vehicle according to the above-described method, it is possible to monitor the medical imaging system from almost arbitrary locations of interest to provide one or more optimal views of a patient and/or other parts of the medical imaging system.

According to an embodiment, navigating the unmanned aerial vehicle to a monitoring location comprises providing information on the magnetic field and steering the unmanned aerial vehicle based on the provided information on the magnetic field. As an example, information on the magnetic field may be provided to the unmanned aerial vehicle by the medical imaging device, or by a user, using the known magnetic fields generated by said medical imaging device. Alternatively, or additionally, the magnetic field may be measured by a magnetic field sensor of the unmanned aerial vehicle. The unmanned aerial vehicle is then steered such that the magnetic fields do not adversely affect the unmanned aerial vehicle.

According to an embodiment, the information on the magnetic field comprises a magnetic field strength and the unmanned aerial vehicle is steered so as to avoid regions where the magnetic field strength exceeds a predetermined threshold. Hence, too strong torques generated by magnetic dipoles of the unmanned aerial vehicle in the magnetic field are avoided.

According to an embodiment, the information on the magnetic field comprises a magnetic field gradient, and the speed of the unmanned aerial vehicle is controlled based on the magnetic field gradient. In particular, the higher the magnetic field gradient, the lower the component of the velocity of the unmanned aerial vehicle along the direction of the magnetic field gradient is chosen. Hence, unwanted induced voltages in electric circuits of the unmanned aerial vehicle are avoided.

According to an embodiment, acquiring monitoring data comprises acquiring monitoring data of a patient, i.e., the unmanned aerial vehicle is used for patient monitoring. As an example, images of the patient may be captured and/or sound from the patient may be recorded. In particular, the patient may be monitored during patient preparation, more particularly to assist the medical staff with patient positioning. Since patient preparation is usually rather short, the unmanned aerial vehicle only requires a small battery, and recharging the battery is fast. In particular, the battery can be easily recharged until the subsequent patient preparation takes place. Alternatively, or additionally, the patient may be monitored during the medical imaging procedure. As examples, the motion and/or vital signs, e.g., breathing or a pulse, of the patient may be detected.

According to an embodiment, acquiring monitoring data comprises acquiring monitoring data of the medical imaging system. In particular, images may be captured, sounds may be recorded, and/or measurements of radiation, magnetic fields, radio frequency fields, temperature and/or humidity may be performed. Said data may be used for real-time monitoring of the medical imaging system or for servicing. As an example, images may be analyzed to determine that state of parts of the medical imaging device, e.g., to detect cracks in coils or to detect loose cabling. As another example, images may be analyzed to detect parts in said images - on the one hand to assure that all parts of the medical imaging system are present, and on the other hand to assure that no foreign parts are present. As yet another example, temperature and humidity may be measured to control a climate control, e.g., air conditioning, accordingly. As yet another example, radiation and/or magnetic fields may be measure to detect possible malfunctioning of the medical imaging device.

According to an embodiment, the monitoring location is a location at or near a part of interest of the medical imaging system, and acquiring monitoring data comprises receiving data from the part of interest via near field communication. For this, the part of interest is configured to communicate via near field communication and the unmanned aerial vehicle may be navigated to all such parts to collect data.

Further details given in the above embodiments of the unmanned aerial vehicle and the monitoring system may be also applied to the embodiments of the method. Vice versa, embodiments of the method may also be applied to embodiments of the unmanned aerial vehicle and to embodiments of the monitoring system.

It shall be understood that a preferred embodiment of the invention can also be any combination of the dependent claims with the respective independent claim. Further, features that have only been described for the apparatus may also apply to the method and vice versa.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig 1 shows a schematic side view of an embodiment of an unmanned aerial vehicle; and
Fig. 2 shows a schematic view of an embodiment of a medical imaging system.

In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

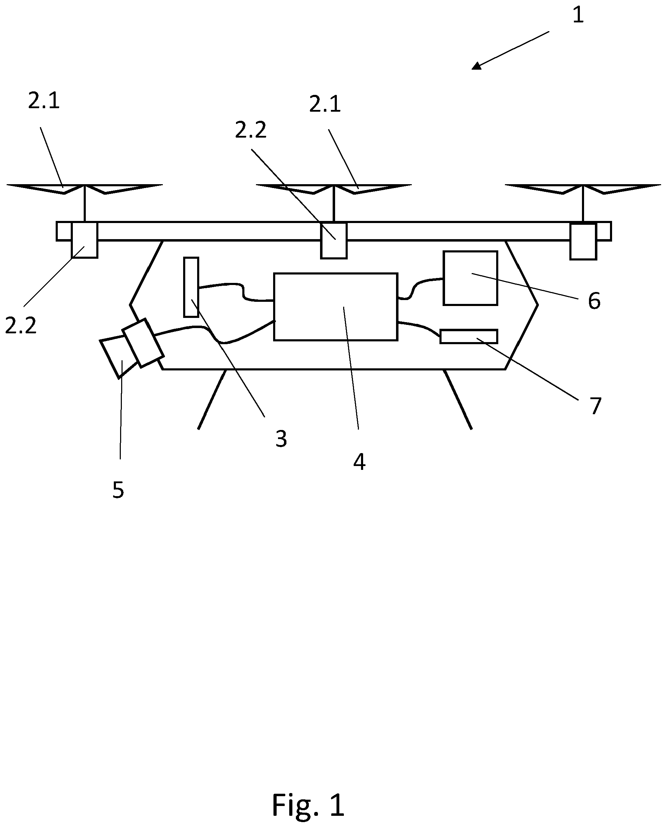

Fig. 1 shows a schematic side view of an unmanned aerial vehicle 1 for monitoring a medical imaging system.

The unmanned aerial vehicle 1 is shown as a quadcopter and comprises an aerodynamic arrangement 2 that includes rotors 2.1 and engines 2.2 associated with these rotors 2.1. However, other unmanned aerial vehicles 1 may also be used, e.g., helicopters or other multicopters.

For navigation of the unmanned aerial vehicle 1, the unmanned aerial vehicle 1 comprises a navigation sensor 3 and a computing unit 4 that is configured to receive output from the navigation sensor 3 and control the aerodynamic arrangement 2 based on said output from the navigation sensor 3. As an example, the navigation sensor 3 may be a radio sensor, receiving radio signals transmitted by radio transmitters located in a medical imaging room, and determining the position of the unmanned aerial vehicle based on time delays of said radio signals. As another example, the navigation sensor 3 may be a camera, using computer vision to detect landmarks in the medical imaging room and determine the position of the unmanned aerial vehicle 1 based on said detected landmarks.

The unmanned aerial vehicle 1 further comprises a monitoring sensor 5 that is configured to acquire monitoring data. Said monitoring sensor 5 is shown as a camera, but other monitoring sensors like microphones, radiation sensors, magnetic field sensors, radio frequency field sensors, thermometers, hygrometers and/or a near field communication sensors may be used in addition or instead. With these monitoring sensors 5, a medical imaging system, such a digital X-ray (DXR) imaging system, a computed tomography (CT) imaging system and/or magnetic resonance imaging (MRI) system may be monitored. In this context, the medical imaging system may comprise a medical imaging device, a medical imaging room, a technical room, auxiliary devices, a patient table and/or a patient.

The monitoring data acquired by the monitoring sensor 5 may be processed by the computing unit 4 to obtain data based on the monitoring data. The monitoring data and/or the data based on the monitoring data may then be stored in a memory 6 of the unmanned aerial vehicle 1. Said memory 6 may be allocated to the computing unit 4 or may be separate from the computing unit 4, as shown in the Fig. Further, the monitoring data and/or the data based on the monitoring data may be wirelessly transmitted using a transmitter 7 of the unmanned aerial vehicle 1. For example, data that is needed for real-time monitoring of the patient may be transmitted using the transmitter 7 whereas data needed for servicing of the medical imaging system may be stored in the memory 6. Alternative embodiments of unmanned aerial vehicles 1 may comprise only the memory 6 but not the transmitter 7 or only the transmitter 7 but not the memory 6.

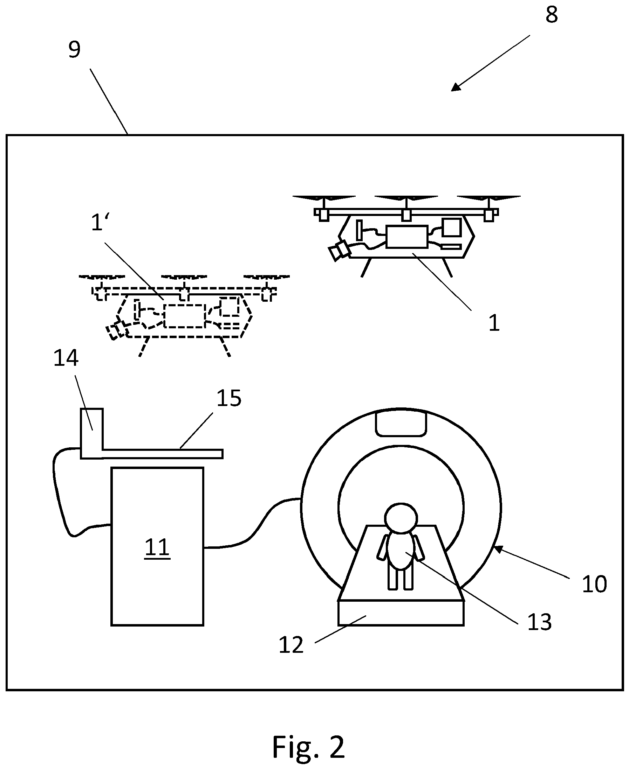

Fig. 2 shows a schematic view of an embodiment of a medical imaging system 8. The medical imaging system 8 may be a digital X-ray (DXR) imaging system, a computed tomography (CT) imaging system and/or magnetic resonance imaging (MRI) system. The medical imaging system 8 encompasses a medical imaging room 9, a medical imaging device 10, a control unit 11 configured to control the medical imaging device 10, a patient table 12, a patient 13.

Further, a monitoring system for monitoring the medical imaging system 8 is shown, comprising the unmanned aerial vehicle 1 and a base station 14. The base station 14 provides a landing platform 15 for the unmanned aerial vehicle 1, and may be configured to provide energy to the unmanned aerial vehicle 1, to receive wireless output from the unmanned aerial vehicle 1 and/or to transfer data from the memory 6 of the unmanned aerial vehicle 1.

The unmanned aerial vehicle 1 may be navigated to a monitoring location where it acquires monitoring data. An alternative monitoring location 1' of the unmanned aerial vehicle 1 is shown in dashed lines. Monitoring the medical imaging system 8 may comprise acquiring monitoring data of the patient 13, e.g., capturing images of the patient 13 and/or recording sounds from the patient 13, more particularly during patient 13 preparation and/or during medical imaging. Monitoring the medical imaging system 8 may further comprise acquiring monitoring data of the medical imaging system 8, more particularly capturing images, recording sounds, and/or performing measurements of radiation, magnetic fields, radio frequency fields, temperature and/or humidity of the medical imaging system.

The above-described monitoring system requires little effort for installation and/or a system upgrade. Further, the unmanned aerial vehicle 1 of the monitoring system can be controlled very precisely and can be steered to almost arbitrary locations of interest to provide one or more optimal views of the patient 13 and/or other parts of the medical imaging system 8.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 1: unmanned aerial vehicle
- 1: alternative position of the unmanned aerial vehicle
- 2: aerodynamic arrangement
- 2.1: rotor
- 2.2: engine
- 3: navigation sensor
- 4: computing unit
- 5: monitoring sensor
- 6: memory
- 7: transmitter
- 8: medical imaging system
- 9: medical imaging room
- 10: medical imaging device
- 11: control unit
- 12: patient table
- 13: patient
- 14: base station
- 15: landing platform

## Claims

1. Unmanned aerial vehicle (1) for monitoring a medical imaging system (8), comprising:
an aerodynamic arrangement (2);
a navigation sensor (3);
a computing unit (4) configured to receive output from the navigation sensor (3) and to control the aerodynamic arrangement (2) based on said output from the navigation sensor (3);
a monitoring sensor (5) configured to acquire monitoring data; and
a memory (6) configured to store the monitoring data or data based on the monitoring data and/or a transmitter (7) configured to wirelessly transmit the monitoring data or data based on the monitoring data.

2. Unmanned aerial vehicle (1) according to claim 1, wherein the aerodynamic arrangement (2) comprises at least one rotating part (2.1) and at least one motor (2.2), more particularly a compressed air motor or an electric motor, e.g., a piezo motor, an ultrasonic motor, or a stepper motor, wherein the motor is configured to actuate the rotating part.

3. Unmanned aerial vehicle (1) according to claim 1 or 2, wherein the navigation sensor (3) is a camera, an infrared sensor, a time-of-flight sensor and/or a radio sensor.

4. Unmanned aerial vehicle (1) according to any one of claims 1 to 3, wherein the monitoring sensor (5) is a camera, a microphone, a radiation sensor, a magnetic field sensor, a radio frequency field sensor, a thermometer, a hygrometer and/or a near field communication sensor.

5. Monitoring system (1; 14) for monitoring a medical imaging system (8), comprising:
an unmanned aerial vehicle (1) according to any one of claims 1 to 4; and
a base station (14).

6. Monitoring system (1; 14) according to claim 5, wherein the base station (14) is configured to provide energy to the unmanned aerial vehicle (1).

7. Monitoring system (1; 14) according to claim 5 or 6, wherein the base station (14) further comprises a receiver configured to receive wireless output from the unmanned aerial vehicle (1).

8. Monitoring system (1; 14) according to any one of claims 5 to 7, wherein the base station (14) is configured to transfer data from the memory (6) of the unmanned aerial vehicle (1) when the unmanned aerial vehicle (1) is docked to the base station (14).

9. Method for operating an unmanned aerial vehicle (1) according to any one of claims 1 to 4, comprising:
taking off;
navigating to a monitoring location;
acquiring monitoring data;
storing the monitoring data or data based on the monitoring data in the memory (6) and/or wirelessly transmitting the monitoring data or data based on the monitoring data; and
landing.

10. Method according to claim 9, wherein navigating to a monitoring location comprises:
providing information on the magnetic field; and
steering the unmanned aerial vehicle (1) based on the provided information on the magnetic field.

11. Method according to claim 10, wherein the information on the magnetic field comprises a magnetic field strength; and the unmanned aerial vehicle (1) is steered so as to avoid regions where the magnetic field strength exceeds a predetermined threshold.

12. Method according to claim 10 or 11, wherein the information on the magnetic field comprises a magnetic field gradient, and the speed of the unmanned aerial vehicle (1) is controlled based on the magnetic field gradient.

13. Method according to any one of claims 9 to 12, wherein acquiring monitoring data comprises acquiring monitoring data of a patient (13), e.g., capturing images of a patient (13) and/or recording sounds from the patient (13), more particularly during patient (13) preparation and/or during medical imaging.

14. Method according to any one of claims 9 to 13, wherein acquiring monitoring data comprises acquiring monitoring data of the medical imaging system (8), more particularly capturing images, recording sounds, and/or performing measurements of radiation, magnetic fields, radio frequency fields, temperature and/or humidity of the medical imaging system (8).

15. Method according to any one of claim 9 to 14, wherein the monitoring location is a location at or near a part of interest of the medical imaging system (8), and acquiring monitoring data comprises receiving data from the part of interest via near field communication.
